# EUROPÄISCHE PATENTSCHRIFT

(11) **EP 0 603 672 B1**
(45) Veröffentlichungstag und Bekanntmachung des Hinweises auf die Patenterteilung: **15.03.2000**
(21) Anmeldenummer: 93119921.0
(22) Anmeldetag: 10.12.1993
(51) Int. Cl.: C12N 15/09, C07K 2/00

(54) **Verfahren zur Herstellung von rekombinanten und synthetischen Peptiden und deren Verwendung**
Process for the production of recombinant and synthetic peptides and their use
Procédé de production de peptides recombinants et synthétiques et leur utilisation

(30) Priorität: 23.12.1992 DE 4243770
(43) Veröffentlichungstag der Anmeldung: 29.06.1994
(73) Patentinhaber: Dade Behring Marburg GmbH, 35001 Marburg (DE)
(72) Erfinder: Grundmann, Ulrich, D-35094 Lahntal (DE); Wissel, Thomas, D-35094 Lahntal (DE); Zettlmeissl, Gerd, D-35083 Wetter (DE)

(56) Entgegenhaltungen:
- WO-A-91/17271
- WO-A-92/00091
- NATURE, Band 354, Nr. 6348, 7. November 1991 K.S. LAM et al. "A new type of synthetic peptide library for identifying ligand- binding activity" Seiten 82-84
- SCIENCE, Band 249, 27. Juli 1990 J.J. DEVLIN et al. "Random Peptide Libraries: A Source of Specific Protein Binding Molecules" Seiten 404-406
- SCIENCE, Band 249, 27. Juli 1990 J.K. SCOTT et al. "Searching for Peptide Ligands with an Epitope Library" Seiten 386-390

## Beschreibung

Die Erfindung betrifft ein Verfahren zur Herstellung von Peptiden, die an einen vorgegebenen, spezifischen Bindungspartner binden, das die Erstellung einer Nukleotid-Bank, Transformation von Mikroorganismen, Kultivierung der transformierten Mikroorganismen und Selektierung der Mikroorganismen, die die gesuchten Peptide exprimieren, einschließt sowie die Verwendung solcher Peptide.

Eine Vielzahl wichtiger Agentien reagiert als Bindungspartner in Rezeptor-Ligand-Wechselwirkungen, die von besonderer therapeutischer und diagnostischer Bedeutung sind. Als Beispiel seien Inhibitoren in proteolytischen Enzymkaskaden, Modulatoren des Immunsystems, Liganden für Hormonrezeptoren, Nukleinsäuren bindende Proteine und Bindungspartner in Antikörper-Antigen-Reaktionen erwähnt.

Häufig ist es von Interesse, die Bindungsstellen von Rezeptor und Ligand zu identifizieren und zu charakterisieren und/oder einen der Bindungspartner durch eine andere Verbindung, vorzugsweise eine niedermolekulare Verbindung, zu ersetzen, die in ähnlicher Weise mit dem anderen Bindungspartner reagiert.

In vielen Fällen ist es wünschenswert, als niedermolekularen Bindungspartner ein synthetisches Peptid einzusetzen, das mit geringem Aufwand und geringen Kosten in großen Mengen und mit hoher Reinheit synthetisierbar ist.

Zur Zeit können in einigen Fällen Strukturen, die von Proteinen bzw. Peptiden dargestellt werden, durch synthetische Peptide ersetzt werden. Allerdings ist es oft sehr aufwendig, wenn nicht unmöglich, die Struktur oder Aminosäuresequenz zu identifizieren, die einen Beitrag zur biologischen Aktivität liefert oder für sie verantwortlich ist. Selbst wenn es in einigen Fällen gelingt, die Aminosäuresequenz der Bindungsstelle eines Proteins mit einem Rezeptor aufzuklären, wie es z.B. bei einigen Antigen-Antikörperbindungen möglich war (Immunology Today, 10, 266-272), ist es nicht vorhersehbar, ob ein synthetisches Peptid, welches die Aminosäuresequenz des Antigens im Bereich der Bindungsstelle umfaßt, in ähnlicher Weise mit dem Antikörper reagiert, da es in Lösung oder auf einer Festphase unter Umständen eine von der Antigenstruktur völlig verschiedene Struktur aufweist und deshalb nicht mit dem entsprechenden Antikörper reagiert.

Es sind empirische Methoden bekannt, die es ermöglichen, bindungsaktive Strukturen zu identifizieren und zu charakterisieren, die in ähnlicher Weise wie die entsprechenden nativen Liganden mit dem ausgewählten Bindungspartner reagieren. Diese Strukturen können dann als rekombinantes oder synthetisches Peptid hergestellt und eingesetzt werden.

Mit Hilfe von molekularbiologischen Methoden, wie in den Patentanmeldungen WO 91/17271, WO 91/19818 sowie in der Literatur von Scott et al., Science 249, 386-390 (1990), Devlin et al., Science 249, 404-406 (1990), Cwirla et al., Proc.Natl.Acad.Sci. USA 87, 6378-6382 (1990) und Felici et al., J. Mol. Biol. (1991) 222, 301-310 beschrieben und chemischen Methoden, wie in der Patentanmeldung WO 92/00091 und von Lam et al., Nature 354, 82-84 (1991) beschrieben, wurden Peptid-Banken hergestellt. Sie stellen umfangreiche Pluralitäten von Peptid-Oligomeren als Bindungspartner bereit, wobei die Pluralität durch Permutationen der 20 Aminosäuren an 5 - 15 Positionen eines Peptids erreicht wird. Die Zahl der theoretisch möglichen Kombinationen bei Permutationen von 5 - 15 Aminosäuren liegt zwischen 3,2 x 10⁶ und 3,3 x 10¹⁹. Die in der Literatur beschriebenen Peptid-Banken umfassen ca. 2,5 x 10⁶ bis 3 x 10⁸ verschiedene Peptide.

Molekularbiologische Verfahren zur Herstellung von Peptid-Banken (i.e. Scott et al., s.o.), beruhen auf der Rekombination von Phagen-DNA mit Oligonukleotiden, die stochastisch verteilte bzw. zufällig variierte Nukleotidsequenzen an 15 bis 60 Positionen enthalten. Wirts-Bakterien werden dann mit der rekombinanten Phagen-DNA transformiert. In den beschriebenen Fällen werden filamentöse Phagen (z.B. M13, fd, f1) eingesetzt. Hierzu werden die Oligonukleotide in Phagen-Gene eingefügt, die Hüll- bzw. Rezeptorproteine wie das pVIII (i. e. Felici et al. s. o.) und das pIII (i.e. Scott et al. s.o.) auf der Oberfläche der Phagen codieren. Die stochastisch verteilten Nukleotidsequenzen werden damit in stochastisch verteilte Aminosäuresequenzen translatiert und auf der Oberfläche der Phagen präsentiert.

Der Einsatz von Phagen für die Herstellung von Peptid-Banken hat z. B. den Nachteil, daß Phagen Bakterien als Wirt benötigen und auch die Anzahl der Kopien des rekombinanten Proteins auf der Oberfläche eines Phagen relativ gering ist.

Ein zur Identifizierung der gesuchten Peptide geeignetes Screeningverfahren muß in der Lage sein, etwa von 3 x 10⁶ bis 4 x 10¹⁹ Verbindungen (s.o.) diejenige zu identifizieren, die mit dem eingesetzten Rezeptor spezifisch reagiert.

Somit sind die Anforderungen an Screeningmethoden außerordentlich hoch und benötigen eine besondere Vorgehensweise.

Das Screening, der im Stand der Technik beschriebenen Phagen-Peptid-Banken und somit die Identifizierung der bindenden Proteine wird nach einem Verfahren durchgeführt, das von Parmely et al. (Gene 73, 305-318 (1988)) wie folgt beschrieben wurde:

Die rekombinanten Phagen werden mit dem auf Petrischalen immobilisierten Rezeptor inkubiert. Nicht angebundene Phagen werden durch Waschen entfernt. Gebundene Phagen werden in einem zweiten Schritt durch Behandlung mit sauren Puffern oder SH-Reagenzien abgelöst und in Nährmedium amplifiziert. Der Reinigungsschritt muß solange wiederholt werden (3-4 mal), bis eine genügend hohe Anreicherung von spezifisch bindenden Phagen erreicht wird. Die Nukleotidsequenz der Phagen wird dann im Bereich der eingefügten Oligonukelotide bestimmt und die Aminosäuresequenz des rezeptor-bindenden Peptids abgeleitet. Das Peptid kann danach chemisch synthetisiert werden.

Nachteilig wirkt sich bei diesem Verfahren allerdings aus, daß die gebundenen Phagen zunächst von dem Träger abgelöst werden müssen, um sie anschließend über Infektion in E.coli zu amplifizieren. Je nach Wahl der Ablösebedingungen besteht dabei die Gefahr, daß entweder die abgelösten Phagen so stark geschädigt werden, daß sie anschließend nicht mehr amplifiziert werden können, oder daß hochaffine Phagen - und damit qualitativ besonders wichtige - nicht abgelöst werden und somit verloren gehen.

Aufgabe der vorliegenden Erfindung war es daher, ein Verfahren zur Verfügung zu stellen, das es ermöglicht, die gewünschten Peptide in hoher quantitativer und qualitativer Ausbeute zu gewinnen.

Gelöst wird diese Aufgabe durch ein Verfahren, in dem eine Vielzahl von Peptiden direkt an der Oberfläche von geeigneten Mikroorganismen in einer Weise exprimiert werden, die es ermöglicht, daß sie Rezeptoren direkt zugänglich sind und mit Hilfe von geeigneten Screeningverfahren identifiziert und isoliert werden können. Die Pluralität der Peptid-Bank sollte dabei größer als 10⁶ sein.

Gegenstand der Erfindung ist daher ein Verfahren zur Herstellung von Peptiden, die an einen vorgegebenen, spezifischen Bindungspartner binden, das folgende Schritte einschließt:
a) Isolierung oder Synthese von Oligonukleotiden, die Bereiche enthalten, in denen die Nukleotidsequenzen stochastisch variieren,
b) Rekombination der Oligonukleotide mit Vektormolekülen in der Weise, daß die Oligonukleotide in ein vektorcodiertes Gen, das ein Protein der äußeren Hülle von Mikroorganismen exprimiert, insertiert werden, wobei je Vektormolekül ein Oligonukleotid eingefügt wird und so eine Vielzahl von Vektormolekülen entsteht, die sich in diesem Bereich in ihrer Nukleotidsequenz unterscheiden (Nukleotid-Bank),
c) Transformation der Mikroorganismen mit den rekombinanten Vektormolekülen,
d) Kultivierung der transformierten Mikroorganismen unter Bedingungen, die es ermöglichen, daß die rekombinanten DNA-Sequenzen exprimiert und die korrespondierenden, rekombinanten Proteine auf der Oberfläche der Mikroorganismen präsentiert werden,
e) Inkubation der Mikroorganismen mit dem an eine Festphase gebundenen, vorgegebenen, spezifischen Bindungspartner, wobei die Mikroorganismen gebunden werden, die das gesuchte Peptid exprimiert haben,
f) Entfernung der nichtgebundenen Mikroorganismen,
g) Vermehrung der gebundenen Mikroorganismen,
h) Vereinzelung und Isolierung von gebundenen Mikroorganismen,
i) Bestimmung der stochastisch variierten Aminosäuresequenzen durch Sequenzierung der korrespondierenden DNA-Sequenzen oder durch Peptidsequenzierung,
j) Gewinnung der gesuchten Peptide durch Isolierung der rekombinanten Proteine aus den Mikroorganismen oder chemische Synthese der entsprechenden Peptide,
   wobei die Mikroorganismen aus der Gruppe der Bakterien und Säugetierzellen ausgewählt werden, vorteilhafterweise wird E. coli als Mikroorganismus verwendet.

Es ist dem Fachmann bekannt, daß bei der unter Schritt b) angeführten Rekombination auch leere Vektoren oder Vektoren mit mehreren Oligonukleotiden erhalten werden können, wobei es unter Umständen vorteilhaft sein kann, gezielt Vektoren mit mehreren Oligonukleotiden herzustellen.

Bevorzugt ist das Verfahren, worin die unter Schritten e - g) isolierten Organismen erneut mit dem Bindungspartner in Kontakt gebracht werden und der Selektions- und Amplifikationsschritt wiederholt wird.

Bevorzugt ist außerdem das Verfahren, worin die rekombinanten Proteine auf der Oberfläche der Organismen und der ausgewählte Rezeptor in Konzentrationen vorliegen, daß nur monovalente Bindungsreaktionen möglich sind.

Besonders vorteilhaft ist das Verfahren, worin die Festphase in Schritt e) partikulär ist, ganz besonders vorteilhaft ist eine magnetisch anziehbare Festphase.

Bevorzugt ist das Verfahren, worin der Vektor ein Plasmid ist.

Bevorzugt ist auch das Verfahren, worin die Nukleotide Kodons enthalten, die durch (NNK)ₓ oder (NNS)ₓ repräsentiert werden, wobei X die Nukleotide A, C, G, T, K die Nukleotide G, T und S die Nukleotide G, C darstellen, x eine Zahl von 4 bis 25, bevorzugterweise 6 ist.

Bevorzugt ist darüberhinaus das Verfahren, worin S = 8 ist und die Nukleotide nur für einen Bruchteil der möglichen Oktapeptide codieren.

Gegenstand der Erfindung ist auch das Verfahren, worin die synthetischen Oligonukleotide eine Serie von Kodons enthalten, die zufällige Kombinationen von Aminosäuren codieren und im 5'- und 3' Bereich der Kodons Sequenzen enthalten, die ebenfalls nicht identisch mit Hüllprotein-Sequenzen sind und die bevorzugterweise für die Antikörperregionen codieren.

Gegenstand der Erfindung ist ferner das Verfahren, worin die synthetisierten Oligonukleotide eine Serie von Kodons enthalten, die zufällige Kombinationen von Aminosäuren codieren und im 5'- und 3' Bereich der Kodons Sequenzen enthalten, die für einen Spacer, der vorteilhafterweise aus Prolinresten besteht, kodieren.

Gegenstand der Erfindung ist auch das Verfahren, worin die Mikroorganismen durch Elektroporation transformiert werden.

Gegenstand der Erfindung ist außerdem das Verfahren, worin die Nukleotid-Bank mindestens 10⁶, vorteilhafterweise 10⁸ Mitglieder umfaßt.

Gegenstand der Erfindung sind auch Peptide mit den Aminosäuresequenzen der Peptide SEQ ID No: 4-17, die nach mindestens einem der erwähnten Verfahren hergestellt werden.

Gegenstand der Erfindung sind ferner Antikörper, die unter Verwendung von solchen Peptiden, die gegebenenfalls an Trägerproteine gekoppelt sind, erzeugt werden.

Gegenstand der Erfindung ist weiterhin ein Verfahren, das es ermöglicht, eine große Vielzahl von Mikroorganismen hinsichtlich ihres Bindungsvermögens an einen ausgewählten Rezeptor gleichzeitig zu untersuchen (Screening).

Das erfindungsgemäße Screeningverfahren ist u. a. einfacher, schneller und effizienter als die bekannten Verfahren. Eine wesentliche Vereinfachung und Beschleunigung besteht darin, daß ein Ablösen der Mikroorganismen von der Festphase nicht mehr erforderlich ist. Gleichzeitig wird es dadurch möglich, auch hochaffine Peptide zu identifizieren und zu isolieren. Eine weitere Beschleunigung und Vereinfachung der Prozedur konnte durch die Verwendung einer magnetisch anziehbaren, mikropartikulären Festphase erreicht werden.

Überraschenderweise konnte gezeigt werden, daß es möglich ist, eine Peptid-Bank von ausreichender Komplexität direkt in E.coli anzulegen, so daß sich die Verwendung von Phagen und die Benutzung eines zusätzlichen Wirts erübrigt.

Beispielsweise wurden Oligonukleotide mit einem Bereich von stochastisch verteilten Nukleotiden hergestellt. Dieser Bereich kann 12, 18, 24, 30 oder mehr Nukleotide umfassen, bevorzugt ist ein Bereich von 18-45 Nukleotiden. In der vorliegenden Erfindung wurde ein Bereich von 18 Nukleotiden gewählt, der entsprechend 6 Aminosäuren in zufälligen Kombinationen kodiert.

Die so hergestellte DNA wurde mit dem Plasmidvektor pHS164-L (EP 0 335 737 A2) rekombiniert, der das "outer membrane Protein A" (OmpA) codiert. Die variable DNA wurde in das ompA-Gen eingefügt. Nach Transformation in E.coli wird das OmpA mit den stochastisch permutierten Aminosäuren auf der E.coli-Hülle präsentiert und ist somit Rezeptoren zugänglich.

Die Präsentation der stochastisch variierten Aminosäuren kann in einer bestimmten Region des OmpA-Proteins so erfolgen, daß in Kombination mit der hier vorgestellten neuen Screeningmethode nützliche Peptidsequenzen identifiziert werden konnten.

OmpA besteht aus 325 Aminosäuren und ist ein sehr häufig vorkommendes Protein der äußeren E.coli Membran. Es dient einigen Phagen und Colicinen als externe Rezeptorstruktur während der Infektion bzw. Intoxikation. Das OmpA enthält vier Regionen mit 10-13 überwiegend hydrophilen Aminosäuren. Diese werden durch Bereiche, die aus 2 x 15 - 17 überwiegend hydrophoben Aminosäuren bestehen, getrennt. Obwohl die dreidimensionale Struktur des OmpA noch nicht bestimmt wurde, haben spektroskopische Messungen ein Modell unterstützt, das das OmpA als ein Protein beschreibt, dessen N-terminale-Hälfte aus 8 Transmembranbereichen mit β-Struktur besteht. Die 4 hydrophilen Bereiche um die Aminosäuren 25, 70, 110 und 154 sind danach an der Bakterien-Oberfläche exponiert.

Pistor und Hobom (Klin. Wochenschr 1988, 66, 110-116) konnten zeigen, daß im Bereich um die Aminosäure 110 Fremdproteinsequenzen in das OmpA eingefügt werden können, die dann selbst ebenfalls an der Oberfläche der E.coli exprimiert werden und die Struktur der OmpA-Sequenzen nicht wesentlich verändern.

Die Präsentation von Hexapeptiden als Bestandteile des äußeren Membranproteins OmpA weist eine Reihe von Vorteilen auf, wie z.B. die einfache Anzucht von Bakterien gegenüber den bisher gebräuchlichen Phagen sowie eine wesentlich höhere Kopienzahl des äußeren Membranproteins pro Zelle.
Das hier eingesetzte Screeningverfahren beruht auf der Expression von Peptiden mit stochastisch permutierten Aminosäuren auf der Oberfläche von Bakterien, derart, daß die Peptide eine Bindung mit Rezeptoren eingehen können. Die Rezeptoren werden auf eine partikuläre, bevorzugterweise magnetisch anziehbare Festphase gebunden. Die Pluralität der Bakterien wird mit der Festphase in Kontakt gebracht. Nach Ablauf der Rezeptor-Ligandreaktion werden die spezifisch gebundenen Bakterien von den ungebundenen abgetrennt. Überraschenderweise konnten die spezifisch gebundenen Bakterien ohne Ablösung von der Festphase weiter vermehrt werden. Ein weiterer Vorteil liegt in der kürzeren Reaktionszeit bei Verwendung einer mikropartikulären festen Phase.

Das im folgenden beschriebene Anwendungsbeispiel beschreibt ein bevorzugtes Verfahren zur Herstellung und zum Screening von Peptid-Banken.

Die Vorteile und Anwendungsmöglichkeiten der Verfahren, die Gegenstand dieser Erfindung sind, werden im folgenden am Beispiel einer Antikörper-Antigen-Bindung verdeutlicht. Dieses Beispiel ist nicht ausschließend zu verstehen, da der Begriff Antikörper-Antigen-Bindung im Sinne der Erfindung mit dem Begriff Rezeptor-Ligand-Wechselwirkung synonym verwendet werden kann.

Das Verfahren ist in der Lage, Pluralitäten von Verbindungen in einer Komplexität darzustellen, die es ermöglicht, Liganden für jeden beliebigen Rezeptor zu isolieren, ohne Informationen über die Existenz oder Natur des natürlichen Liganden zu besitzen.

Es können darüberhinaus Liganden mit unterschiedlichen Affinitäten für jeden immobilisierbaren Rezeptor isoliert werden, von denen dann diejenigen mit den besten Eigenschaften ausgewählt werden können.

Ebenso kann ein Epitop-Mapping wesentlich effizienter durchgeführt werden, als mit chemischen Methoden. So können neben monoklonalen Antikörpern auch Gemische von Antikörpern, wie polyklonale Antikörper, analysiert werden. Sind z.B. die Primärstrukturen des Antigens oder der Antigene bekannt, können viele Epitope, gegen die die polyklonalen Antikörper gerichtet sind, in einem Arbeitsgang identifiziert werden.

### Beispiele

### Beispiel 1

### Konstruktion einer Hexapeptid-Epitop-Bank in Escherichia coli (E.coli)

Zur Präsentation von Hexapeptidmolekülen auf der Oberfläche von E.coli wurde der Plasmidvektor pHS164-L (EP 0 355 737 A2) gewählt, der das äußere Membranprotein A (ompA) gramnegativer Bakterien kodiert und eine Polyklonierungsstelle aufweist. In die Restriktionsstellen ClaI und XmaI wurden Oligonukleotide inseriert, die alle Hexapeptidkombinationen kodieren sollten. Sie wurden seitlich durch fünf weitere, hydrophile Aminosäuren, wie z. B. Prolin und Glycin flankiert, die, mit einer Stielregion vergleichbar, die Hexapeptidregion besser auf der Bakterienoberfläche präsentieren sollen. Damit ergab sich folgende, zu synthetisierende Nukleinsäuresequenz:

SEQ ID NO: 1

5' CG CCA GGA CCC CCG CCT XXN XXN XXN XXN XXN XXN CCT CCT
CCG CCA CC 3'

"N" symbolisiert dabei alle vier Kodierungsmöglichkeiten (A,C,G,T) und "K" nur G und T. Durch diese Kodierung werden alle zwanzig, natürlich vorkommenden Aminosäuren zufallsmäßig vorgegeben. Um einen DNA-Doppelstrang in den mit ClaI/XmaI geöffneten Vektor einligieren zu können, wurde der oben gezeigte Einzelstrang in seinen Randbereichen, die nur Glycin oder Prolin kodieren, durch zwei weitere, gegenläufige (revers komplementäre) Oligonukleotide teilweise in einen Doppelstrang verwandelt. Hierzu wurden die Oligonukleotide 
SEQ ID NO: 2 3' GGT CCT GGG GGC GGA 5'
SEQ ID NO: 3 3' GGA GGC GGT GGG GCC 5'
 synthetisiert und an den konstant gehaltenen Bereich des DNA-Einzelstrangs hybridisiert. Dadurch kann das in seinen Randbereichen doppelsträngige Oligonukleotid in die ClaI und XmaI Restriktionsstellen des geöffneten Vektors einligiert und in die Wirtszelle auch transformiert werden.

### Durchführung:

Zur Durchführung der DNA-Konstruktion wurde die DNA des Vektors pHS164-L an der multiplen Polyklonierungsstelle mit dem Restriktionsenzym ClaI, das die DNA-Sequenz ^{5'}AT'CGAT^{3'} erkennt, und XmaI, das die DNA-Sequenz ^{5'}C'CCGGG^{3'} erkennt, geöffnet und über Gelelektrophorese gereinigt. In diese Schnittstellen hinein wurde das Oligonukleotid, das potentiell für 64 x 10⁶ Hexapeptide sowie für die benachbarten, zur Stielregion beitragenden Aminosäuren Prolin bzw Glycin kodiert, hineinligiert. Die Ligasereaktion erfolgte mit 10 µg geöffnetem Vektor und 0,154 µg doppelsträngigem, hybridisiertem Oligonukleotid in einem Gesamt-Ligaseansatz von 10 µl bei Raumtemperatur über 24 h. Vor Transformation durch Elektroporation wurde die DNA mittels Centrikon™30 Mikrokonzentratoren (Amicon, Berverly, MA, USA) entsalzt. Die Transformation dieser DNA in die Zellen des Escherichia coli Stammes 490 A, die zuvor über Standardmethoden kompetent gemacht wurden, erfolgte mit 1 µg DNA des Ligaseansatzes in 100µl kompetenter Zellen mittels Elektroporation im Gene Pulser™ (BioRad, Richmond, CA, USA) bei 2,4 kVolt, 400 Ohm 25 µFD in Küvetten mit dem Maß 0,2 cm. Zehn Transformationsansätze, wie oben beschrieben, wurden gesammelt, vereinigt und ergaben schließlich 3 x 10⁸ transformierte Zellen. Durch Restriktionsverdau von verschiedenen Zellklonen konnte gezeigt werden, daß 42 % der transformierten Zellen die für ein Hexapeptid kodierende DNA aufgenommen hatten. Somit betrug die durchschnittliche Transformationsrate des rekombinanten, allein für die Hexapeptide codierenden Plasmids etwa 1,26 x 10⁷/µg.

Alle theoretisch möglichen Hexapeptidkombinationen waren somit zweimal repräsentiert. Die Epitop-Bank wurde in 1 ml Kulturen eingeteilt und mit 10 % Glycerin bei -80 °C eingefroren.

### Beispiel 2

### Kopplung von Magnetpartikeln M450 mit Antikörpern

Am Beispiel von drei monoklonalen Antikörpern, die gegen verschiedene virale Peptide gerichtet sind, kann nachfolgend gezeigt werden, daß die jeweiligen Antikörper aus der bakteriellen Epitop-Bank, in der mehr als 1,2 x 10⁸ Hexapeptide präsentiert werden, bestimmte Epitope herausfinden.
- ad a:: MAK 144/158 gerichtet gegen das HBeAG des Hepatitis B Virus (Molecular Immunology 28, 1991, 719-726)
- ad b:: MAK 91-195/039 gerichtet gegen ein synthetisches Peptid, das die Aminosäuren 120-130 des gag-Proteins des "Human adult T-cell leukemia virus" enthält. (Proc. Natl. Acad. Sci USA 80, 1983, 3518-3622)
- ad c:: MAK-87-55/02(2 gerichtet gegen ein Epitop des pp150 Proteins des Cytomegalovirus (EPA 0 534 102 A1).

### Durchführung:

ad 1: Zum Screening der Epitop-Bank mit dem monoklonalen Antikörper MAK 144/158 wurde dieser mit Magnetpartikeln M450 (Magnetobeads M450, Dynabeads™, Dynal A.S., Norwegen) gekoppelt. Auf ein Volumen von 1ml in 40 mM Boratpuffer, pH = 9,5, entfielen dabei 250 µl Magnetpartikel und 75 µg Antikörper. Dieser Ansatz wurde über Nacht bei Raumtemperatur geschüttelt und anschließend 3 mal mit Lagerpuffer (50 mM 2-[N-Cyclohexylamino]-Ethansulfonsäure (kurz CHES), 0,01 % (w/v) Na-Azid) in der magnetischen Trenneinheit gewaschen. Die an die Magnetpartikel gekoppelten Antikörper wurden schließlich in 1ml Lagerpuffer bei 4 °C aufbewahrt. Sie wurden unmittelbar vor Benutzung (Screening) dreimal mit LB-Medium gewaschen.

ad 2 und 3: Zum Screening der Epitopbank mit den monoklonalen Antikörpern MAK 91-195/039 und MAK 87-55/02/2 wurden diese mit Magnetpartikeln M1-070/40 (Lot. 383) (Estapor ^{R} Microsperes Rhône-Poulenc, Frankreich) gekoppelt. Es wurden 500 µl Magnetpartikel mit 3x 5ml Kopplungspuffer (100 mM N-2-hydroxyethylpiperazine-N'-2-ethane-sulfonic acid, - kurz HEPES -, pH 4,7) in einer magnetischen Trenneinheit gewaschen und in 8 ml Kopplungspuffer aufgenommen. Unter Schütteln wurden 1,5mg Antikörper und 2ml einer 1-Ethyl-3(3-dimethylamino-propyl)carbodiimide HCl-Lösung (2mg/ml) zugegeben und auf ein Volumen von 20 ml mit Kopplungspuffer eingestellt. Die Suspension wurde 16 Stunden unter Schütteln bei 4°C inkubiert. Die Magnetpartikel wurden anschließend 3 mal mit Lagerpuffer (50mM 2[N-Cyclohexylaminol]-Ethansulfonsäure - kurz Ches -, 0,0%Na-azid) in der magnetischen Trenneinheit gewaschen. Die an die Magnetpartikel gekoppelten Antikörper wurden schlißlich in 1ml Lagerpuffer bei 4°C aufbewahrt. Sie wurden unmittelbar vor Benutzung (Screening) dreimal mit LB-Medium gewaschen.

### Beispiel 3

### Screening der Epitop-Bank

### Durchführung:

1 ml einer bei -80 °C gelagerten Kultur der Epitop-Bank wurde aufgetaut und in 200 ml L-Broth Medium/50 µg/ml Ampicillin über Nacht bei 37 °C inkubiert. Am Morgen wurde 1ml dieser Kultur erneut zu 200 ml L-Broth/Ampicillin zugegeben und bei 37 °C bis OD_{600 nm} = 0,5 inkubiert. In Gegenwart von 1mM IPTG (Isopropyl-β-thiogalaktosid) wurden die Zellen induziert und 2 h unter gleichen Bedingungen weiterinkubiert.

Zum Screening der Epitop-Bank mit dem monoklonalen Antikörper wurden nunmehr 100 µl der Zellkultur erneut mit 900 µl L-Broth/Ampicillin versetzt und in Gegenwart von 1 % (v/v) Tween20 mit 10 µl der mit Magnetpartikeln gekoppelten Antikörper (75 µg/ml) 1 h unter leichtem Schütteln bei 4 °C inkubiert. Mit Hilfe der magnetischen Trenneinrichtung wurden die Magnetpartikel mitsamt den MAK's und allen daran gebundenen Zellen abgetrennt und noch viermal mit Waschpuffer (10 mM Tris/pH=7,4, 0,9 % (w/v) NaCl, 1 % Tween20) gewaschen und in 200 µl L-Broth/Ampicillin aufgenommen. 10 µl davon wurden auf L-Broth Agar mit Ampicillin ausplattiert und über Nacht bei 37 °C inkubiert. Durchschnittlich wuchsen auf dieser Platte 500-1000 Kolonien heran.

Für ein weiteres Screening dieser Zellen mit den gleichen, an Magnetpartikel gebundenen MAKs wurden die Zellen mit 1 ml L-Broth abgespült und in 300 ml L-Broth/Ampicillin erneut bis OD_{600 nm} = 0,5 bei 37 °C inkubiert. Dann wurden die Zellen wiederum mit 1 mM IPTG induziert; das zweite Screening erfolgte unter den gleichen Bedingungen wie oben beschrieben. Das Screening mit einem MAK wurde insgeamt viermal wiederholt.

### Beispiel 4

### Analyse positiver Klone

### a) Screening mit MAK 144/158 (HBeAg)

Zur Analyse positiver Klone, die aufgrund des Screenings mit dem monoklonalen Antikörper MAK 144/158 isoliert werden konnten, wurden anschließend drei verschiedene Methoden angewandt. In Hemmversuchen wurde geprüft, ob die Bindung der positiven Klone an die mit dem MAK 144/158 gekoppelten Magnetpartikel durch Zugabe freier MAK 144/158 behindert werden konnte. Mit Hilfe der Western-Blot Analyse wurde die Bindung des monoklonalen Antikörpers 144/158 an den im OmpA Protein integrierten Epitop-Bereich untersucht und dann wurde durch Sequenzanalyse die Nukleinsäuresequenz des entsprechenden Epitop-Bereichs bestimmt. Dabei wurde überwiegend ein Peptid identifiziert, das früher schon von Sällberg et al. 1991, Molecular Immunology 28, 719-726, auf herkömmlichem Wege durch "fine mapping" mit der Aminosäuresequenz T-P-P-A-Y-R bestimmt worden war.

### Durchführung:

### Hemmversuche

Einzelklone aus dem Screening wurden über Nacht inkubiert, die Kultur morgens verdünnt und schließlich mit IPTG induziert. Die Zellkultur wurde nun mit L-Broth/Ampicillin 1:100 verdünnt (Gesamtansatz 250 µl) und in Gegenwart von 1% Tween20 und unterschiedlichen Konzentrationen freiem MAK 144/158 (10-100 µg/ml Ansatz) 1h bei 4°C inkubiert. Danach wurden die Magnetpartikel mit dem gebundenen MAK 144/158 zugegeben und nochmals 1h bei 4°C inkubiert. Anschließend wurden die Zellen wieder über die magnetische Trenneinrichtung isoliert und dreimal mit Waschpuffer gewaschen. Die Zellen wurden danach in 500 µl L-Broth/Ampicillin aufgenommen und jeweils 20 µl davon auf L-Broth Agar/Ampicillin ausplattiert. Parallel hierzu erfolgten Versuchsreihen ohne die Zugabe von freiem MAK 144/158 (Positiv-Kontrolle). Außerdem erfolgten Experimente derart, daß Zellen, die mit dem Vektor pHS164-L ohne Inserts transformiert worden waren, in Parallelversuchen mitgeführt wurden (Negativ-Kontrolle). Die Versuche zeigten, daß durch Zugabe von freiem MAK 144/158 zum Reaktionsansatz die Bindung der Zellen an den an die Magnetpartikel gebundenen MAK 144/158 um den Faktor 50 bis 100 vermindert wurde. Im Falle der Positiv-Kontrolle war die Bindung der Zellen an die Magnetpartikel dagegen nicht beeinträchtigt und im Falle der Negativ-Kontrolle kam erwartungsgemäß keine Zellbindung zustande.

### Western-Blot Analyse

Zell-Klone, die durch das Screening mit MAK 144/158 gefunden worden waren und deren Bindung im Hemmversuch durch den freien MAK 144/158 gehemmt werden konnte, wurden im nächsten Schritt einer Western-Blot Analyse unterzogen. Alle Klone zeigten dabei eine positiv reagierende Proteinbande, die der Proteingröße des Mebranprotein OmpA entspricht.

### DNA-Sequenz Analyse

Alle Zell-Klone, die in den vorangegangenen Analysen positiv reagierten, wurden nunmehr einer DNA-Sequenzanalyse unterworfen. Dazu wurde die Vektor-DNA nach herkömmlichen Verfahren als doppelsträngige DNA isoliert und mit Vektor-spezifischen Oligonukleotid-Primern nach Sanger (Sanger et al., 1977, Proc.Natl.Acad.Sci. USA, 74, 5463-5467) sequenziert. Die Analyse zeigte, daß mit einer Ausnahme alle Klone eine DNA-Sequenz enthalten, die die ursprüngliche Peptidsequenz, welche der MAK 144/158 erkennt - nämlich T-P-P-A-Y-R - kodieren. Die entsprechende DNA-Sequenz eines einzigen Zell-Klons wich von dieser ursprünglichen Sequenz ab und zeigte eine Nukleotidfolge, die stattdessen die folgende Aminosäuresequenz SEQ ID NO: 4 kodiert.

SEQ ID NO: 4 Leu Pro Pro Ala Phe Arg

### 4b) Screening mit MAK 91-195/039 (HTLV)

Zur Analyse positiver Klone, die aufgrund des Screenings mit dem monoklonalen Antikörper MAK 91-195/039 isoliert werden konnten, wurden wiederum drei verschiedene Methoden angewandt. In Hemmversuchen wurde geprüft, ob die Bindung der positiven Klone an die mit dem MAK 91-195/039 gekoppelten Magnetpartikel durch Zugabe freier MAK 91-195/039 behindert werden konnte. Mit Hilfe der Western-Blot Analyse wurde die Bindung des monoklonalen Antikörpers 91-195/039 an den im OmpA Protein integrierten Epitop-Bereich untersucht und durch Sequenzanalyse schließlich wurde die Nukleinsäuresequenz des entsprechenden Epitop-Bereichs bestimmt. Dabei wurden ausschließlich Peptide gefunden, deren Sequenz von dem Bereich P Y V E P Y A P Q V L des Immunisierungsantigens abwich (Proc. Natl. Acad. Sci. USA 1983, 80, 3618-3622).

### Durchführung:

### Hemmversuche

Einzelklone aus dem Screening wurden über Nacht inkubiert, die Kultur morgens verdünnt und schließlich mit IPTG induziert. Die Zellkultur wurde nun mit L-Broth/Ampicillin 1:100 verdünnt (Gesamtansatz 250 µl) und in Gegenwart von 1% Tween20 und unterschiedlichen Konzentrationen freiem MAK 91-195/039 (10-100 µg/ml Ansatz) 1h bei 4°C inkubiert. Danach wurden die Magnetpartikel mit dem gebundenen MAK 91-195/039 zugegeben und nochmals 1h bei 4°C inkubiert. Anschließend wurden die Zellen wieder über die magnetische Trenneinrichtung isoliert und dreimal mit Waschpuffer gewaschen. Die Zellen wurden danach in 500 µl L-Broth/Ampicillin aufgenommen und jeweils 20 µl davon auf L-Broth Agar/Ampicillin ausplattiert. Parallel hierzu erfolgten Versuchsreihen ohne die Zugabe von freiem MAK 91-195/039 (Positiv-Kontrolle). Außerdem erfolgten Experimente derart, daß Zellen, die mit dem Vektor pHS164-L ohne Inserts transformiert worden waren, in Parallelversuchen mitgeführt wurden (Negativ-Kontrolle). Die Versuche zeigten, daß durch Zugabe von freiem MAK 91-195/039 zum Reaktionsansatz die Bindung der Zellen an den an die Magnetpartikel gebundenen MAK 91-195/039 um den Faktor 20 bis 30 vermindert wurde. Im Falle der Positiv-Kontrolle war die Bindung der Zellen an die Magnetpartikel dagegen nicht beeinträchtigt und im Falle der Negativ-Kontrolle kam erwartungsgemäß keine Zellbindung zustande.

### Western-Blot Analyse

Zell-Klone, die durch das Screening mit MAK 91-195/039 gefunden worden waren und deren Bindung im Hemmversuch durch den freien MAK 91-195/039 deutlich gehemmt werden konnte, wurden im nächsten Schritt einer Western-Blot Analyse unterzogen. Mit einer Ausnahme zeigten alle Klone dabei eine positiv reagierende Proteinbande, die der Proteingröße des Mebranprotein OmpA entspricht.

### DNA-Sequenz Analyse

Zell-Klone, die durch das Antikörper-Screening gefunden worden waren, wurden nunmehr einer DNA-Sequenzanalyse unterworfen. Dazu wurde die Vektor-DNA nach herkömmlichen Verfahren als doppelsträngige DNA isoliert und mit Vektor-spezifischen Oligonukleotid-Primern nach Sanger (Sanger et al., 1977, Proc.Natl.Acad.Sci. USA, 74, 5463-5467) sequenziert. Die Analyse zeigte, daß ausnahmslos alle Klone eine DNA-Sequenz enthalten, die nicht der Wildtypsequenz Pro-Tyr-Val-Glu-Pro-Thr-Ala-Pro-Gln-Val-Leu entspricht, die der MAK 91-195/039 erkennt. Vielmehr wurden insgesamt 8 verschiedene Peptid-Sequenzen unter den positiven Klonen gefunden, die entweder in der Western-blot Analyse oder im Hemmversuch oder in beiden Ansätzen positiv reagierten. Folgende Peptid-Sequenzen wurden gefunden:

SEQ ID NO: 5 | Phe Leu Phe Pro Thr Ser | Klon 5
SEQ ID NO: 6 | Met Asn Phe Asn Ser Ser | Klon 8
SEQ ID NO: 7 | Ser Leu Ala Ala Thr Trp | Klon 9
SEQ ID NO: 8 | Val Asn Ile Asn Ser Gln | Klon 12
SEQ ID NO: 9 | Val Asn Tyr Asn Ser Ser | Klon 13
SEQ ID NO: 10 | Phe Ile Ala Pro Met Gly | Klon 30
SEQ ID NO: 11 | Tyr Ile Leu Ala Thr Leu | Klon 31
SEQ ID NO: 12 | Tyr Leu Ser Pro Phe Gly | Klon 33

### 4c) Screening mit MAK 87-55/02/2 (CMV)

Zur Analyse positiver Klone, die aufgrund des Screenings mit dem monoklonalen Antikörper MAK 87-55/02/2 isoliert werden konnten, wurden auch drei verschiedene Methoden angewandt. In Hemmversuchen wurde geprüft, ob die Bindung der positiven Klone an die mit dem MAK 87-55/02/2 gekoppelten Magnetpartikel durch Zugabe freier MAK 87-55/02/2 behindert werden konnte. Mit Hilfe der Western-Blot Analyse wurde die Bindung des monoklonalen Antikörpers 87-55/02/2 an den im OmpA Protein integrierten Epitop-Bereich untersucht und durch Sequenzanalyse schließlich wurde die Nukleinsäuresequenz des entsprechenden Epitop-Bereichs bestimmt. Es wurden Peptide gefunden, die der CMV Wildtypsequenz des pp150 von Stüber et al. (EP-A-0 534 102), auf herkömmlichem Wege durch "fine mapping" mit der Aminosäuresequenz Asp-Met-Asn-Pro-Ala-Asn-Trp-Pro-Arg-Glu-Arg-Ala-Trp-Ala-Leu bestimmt.

### Durchführung:

### Hemmversuche

Einzelklone aus dem Screening wurden über Nacht inkubiert, die Kultur morgens verdünnt und schließlich mit IPTG induziert. Die Zellkultur wurde nun mit L-Broth/Ampicillin 1:100 verdünnt (Gesamtansatz 250 µl) und in Gegenwart von 1% Tween20 und unterschiedlichen Konzentrationen freiem MAK 87-55/02/2 (10-100 µg/ml Ansatz) 1h bei 4°C inkubiert. Danach wurden die Magnetpartikel mit dem gebundenen MAK 87-55/02/2 zugegeben und nochmals 1h bei 4°C inkubiert. Anschließend wurden die Zellen wieder über die magnetische Trenneinrichtung isoliert und dreimal mit Waschpuffer gewaschen. Die Zellen wurden danach in 500 µl L-Broth/Ampicillin aufgenommen und jeweils 20 µl davon auf L-Broth Agar/Ampicillin ausplattiert. Parallel hierzu erfolgten Versuchsreihen ohne die Zugabe von freiem MAK 87-55/02/2 (Positiv-Kontrolle). Außerdem erfolgten Experimente derart, daß Zellen, die mit dem Vektor pHS164-L ohne Inserts transformiert worden waren, in Parallelversuchen mitgeführt wurden (Negativ-Kontrolle). Die Versuche zeigten, daß durch Zugabe von freiem MAK 87-55/02/2 zum Reaktionsansatz die Bindung der Zellen an den an die Magnetpartikel gebundenen MAK 87-55/02/2 um den Faktor 20 bis 30 vermindert wurde. Im Falle der Positiv-Kontrolle war die Bindung der Zellen an die Magnetpartikel dagegen nicht beeinträchtigt und im Falle der Negativ-Kontrolle kam erwartungsgemäß keine Zellbindung zustande.

### Western-Blot Analyse

Zell-Klone, die durch das Screening mit MAK 87-55/02/2 gefunden worden waren und deren Bindung im Hemmversuch durch den freien MAK 87-55/02/2 deutlich gehemmt werden konnte, wurden im nächsten Schritt einer Western-Blot Analyse unterzogen. Alle Klone zeigten dabei eine positiv reagierende Proteinbande, die der Proteingröße des Mebranprotein OmpA entspricht.

### DNA-Sequenz Analyse

Alle Zell-Klone, die in den vorangegangenen Analysen positiv reagierten, wurden nunmehr einer DNA-Sequenzanalyse unterworfen. Dazu wurde die Vektor-DNA nach herkömmlichen Verfahren als doppelsträngige DNA isoliert und mit Vektor-spezifischen Oligonukleotid-Primern nach Sanger (Sanger et al., 1977, Proc.Natl.Acad.Sci. USA, 74, 5463-5467) sequenziert. Die Analyse zeigte, daß die positiven Klone eine DNA-Sequenz enthalten, die der ursprünglichen Peptidsequenz im Bereich Asp-Met-Asn-Pro-Ala-Asn-Trp-Pro-Arg sehr nahe kommen. Es sind insgesamt fünf verschiedene, von der Wildtypsequenz abweichende Hexapeptide gefunden worden:

SEQ ID NO: 13 | Leu Val Asn Pro Ala Asn | Klon 4
SEQ ID NO: 14 | Phe Asn Pro Ala Asn Phe | Klon 7
SEQ ID NO: 15 | Asp Arg Asn Pro Ala Asn | Klon 8
SEQ ID NO: 16 | Asp Tyr Asn Ala Ala Asn | Klon 9
SEQ ID NO: 17 | Phe Asn Pro Ala Asn Asn | Klon 11

## Patentansprüche

1. Verfahren zur Herstellung von Peptiden, die an einen vorgegebenen, spezifischen Bindungspartner binden, das folgende Schritte einschließt:
a) Isolierung oder Synthese von Oligonukleotiden, die Bereiche enthalten, in denen die Nukleotidsequenzen stochastisch variieren,
b) Rekombination der Oligonukleotide mit Vektormolekülen in der Weise, daß die Oligonukleotide in ein vektor-codiertes Gen, das ein Protein der äußeren Hülle von Mikroorganismen exprimiert, insertiert werden, wobei je Vektormolekül ein Oligonukleotid eingefügt wird und so eine Vielzahl von Vektormolekülen entsteht, die sich in diesem Bereich in ihrer Nukleotidsequenz unterscheiden (Oligo-Nukleotid-Bank),
c) Transformation der Mikroorganismen mit den rekombinanten Vektormolekülen,
d) Kultivierung der transformierten Mikroorganismen unter Bedingungen, die es ermöglichen, daß die rekombinanten DNA-Sequenzen exprimiert und die korrespondierenden, rekombinanten Proteine auf der Oberfläche der Mikroorganismen präsentiert werden,
e) Inkubation der Mikroorganismen mit dem an eine Festphase gebundenen, vorgegebenen, spezifischen Bindungspartner, wobei die Mikroorganismen gebunden werden, die das gesuchte Peptid exprimiert haben,
f) Entfernung der nichtgebundenen Mikroorganismen,
g) Vermehrung der gebundenen Mikroorganismen,
h) Vereinzelung und Isolierung von gebundenen Mikroorganismen,
i) Bestimmung der stochastisch variierten Aminosäuresequenzen durch Sequenzierung der korrespondierenden DNA-Sequenzen oder durch Peptidsequenzierung,
j) Gewinnung der gesuchten Peptide durch Isolierung der rekombinanten Proteine aus den Mikroorganismen oder chemische Synthese der entsprechenden Peptide
dadurch gekennzeichnet, daß die Mikroorganismen aus der Gruppe der Bakterien und Säugetierzellen ausgewählt werden.

2. Verfahren nach Anspruch 1, worin die unter Schritten e - g) isolierten Organismen erneut mit dem Bindungspartner in Kontakt gebracht werden und der Selektions- und Amplifikationsschritt wiederholt wird.

3. Verfahren nach Anspruch 1, worin die rekombinanten Proteine auf der Oberfläche der Organismen und der ausgewählte Rezeptor in Konzentrationen vorliegen, daß nur monovalente Bindungsreaktionen möglich sind.

4. Verfahren nach Anspruch 1, worin der Mikroorganismus ein Bakterium ist.

5. Verfahren nach Anspruch 1, wobei der Mikroorganismus eine suspendierte Säugetierzelle ist.

6. Verfahren nach Anspruch 4, worin der Mikroorganismus ein E.coli ist.

7. Verfahren nach Anspruch 4 und 6, worin das Hüllprotein OmpA ist.

8. Verfahren nach Anspruch 1, worin die Festphase in Schritt e) partikulär ist.

9. Verfahren nach Anspruch 8, worin die Festphase magnetisch anziehbar ist.

10. Verfahren nach Anspruch 1, worin der Vektor ein Plasmid ist.

11. Verfahren nach Anspruch 1, worin die Nukleotide Kodons enthalten, die durch (NNK)ₓ repräsentiert werden, wobei N die Nukleotide A, C, G, T und K die Nukleotide G, T darstellen sowie X eine Zahl von 4 bis 25 ist.

12. Verfahren nach Anspruch 11, worin X = 6 ist.

13. Verfahren nach Anspruch 11, worin X = 8 ist und die Nukleotide nur für einen Bruchteil der möglichen Oktapeptide codieren.

14. Verfahren nach Anspruch 11, worin X = 10 ist und die Nukleotide nur für einen Bruchteil der möglichen Dekapeptide codieren.

15. Verfahren nach Anspruch 1, worin die Oligonukleotide eine Serie von Kodons enthalten die zufällige Kombinationen von Aminosäuren codieren und im 5'- und 3' Bereich des Kodons Sequenzen enthalten, die ebenfalls nicht identisch mit Hüllprotein-Sequenzen sind.

16. Verfahren nach Anspruch 1, worin die synthetisierten Oligonukleotide eine Serie von Kodons enthalten die zufällige Kombinationen von Aminosäuren codieren und im 5'- und 3' Bereich der Kodons Sequenzen enthalten, die Antikörperregionen codieren.

17. Verfahren nach Anspruch 1, worin die synthetisierten Oligonukleotide eine Serie von Kodons enthalten die zufällige Kombinationen von Aminosäuren codieren und im 5'- und 3' Bereich der Kodons Sequenzen enthalten, die für einen Spacer kodieren.

18. Verfahren nach Anspruch 17, worin der Spacer Prolinreste enthält.

19. Verfahren nach Anspruch 1, worin die Mikroorganismen durch Elektroporation transformiert werden.

20. Verfahren nach Anspruch 1, worin die Nukleotid-Bank mindestens 10⁶ umfaßt.

21. Peptide mit den Aminosäuresequenzen der Peptide SEQ ID NO: 4-17, die nach mindestens einem der Verfahren nach Anspruch 1 - 21 hergestellt werden.

22. Antikörper, die unter Verwendung von Peptiden nach Anspruch 21 gegebenenfalls an Trägerproteine gekoppelt sind, erzeugt werden.

## Claims

1. A process for preparing peptides which bind to a predetermined, specific binding partner, which process includes the following steps:
a) isolation or synthesis of oligonucleotides which contain regions in which the nucleotide sequences vary stochastically,
b) recombination of the oligonucleotides with vector molecules in such a way that the oligonucleotides are inserted into a vector-encoded gene which expresses a protein of the outer coat of microorganisms, an oligonucleotide being inserted into each vector molecule, thereby giving rise to a multiplicity of vector molecules which differ from each other in this region of their nucleotide sequence (oligonucleotide library),
c) transformation of the microorganisms with the recombinant vector molecules,
d) cultivation of the transformed microorganisms under conditions which permit the recombinant DNA sequences to be expressed and the corresponding recombinant proteins to be presented on the surface of the microorganisms,
e) incubation of the microorganisms with the pre-determined, specific binding partner, which is bound to a solid phase, with those microorganisms being bound which have expressed the sought-after peptide,
f) removal of the unbound microorganisms,
g) replication of the bound microorganisms,
h) cloning and isolation of bound microorganisms
i) determination of the stochastically varied amino acid sequences by sequencing the corresponding DNA sequences or by peptide sequencing,
j) obtaining the sought-after peptides by isolating the recombinant proteins from the microorganisms or by chemical synthesis of the corresponding peptides
wherein the microorganisms are selected from the group comprising bacteria and mammalian cells.

2. The process as claimed in claim 1, wherein the organisms isolated under steps e-g) are brought into contact once again with the binding partner and the selection and amplification steps are repeated.

3. The process as claimed in claim 1, wherein the recombinant proteins on the surface of the organisms and the selected receptor are present in concentrations such that only monovalent binding reactions are possible.

4. The process as claimed in claim 1, wherein the microorganism is a bacterium.

5. The process as claimed in claim 1, wherein the microorganism is a mammalian cell in suspension.

6. The process as claimed in claim 4, wherein the microorganism is an E.coli

7. The process as claimed in claims 4 and 6, wherein the coat protein is OmpA.

8. The process as claimed in claim 1, wherein the solid phase in step e) is particulate.

9. The process as claimed in claim 8, wherein the solid phase is magnetically attractable.

10. The process as claimed in claim 1, wherein the vector is a plasmid.

11. The process as claimed in claim 1, wherein the nucleotides contain codons which are represented by (NNK)ₛ, wherein N represents the nucleotides A, C, G and T and K represents the nucleotides G and T, and X is a number from 4 to 25.

12. The process as claimed in claim 11, wherein X = 6.

13. The process as claimed in claim 11, wherein X = 8 and the nucleotides encode only a fraction of the possible octapeptides.

14. The process as claimed in claim 11, wherein X = 10 and the nucleotides encode only a fraction of the possible decapeptides.

15. The process as claimed in claim 1, wherein the oligonucleotides contain a series of codons which encode random combinations of amino acids and, in the 5' and 3' regions of the codon, contain sequences which likewise are not identical to coat protein sequences.

16. The process as claimed in claim 1, wherein the synthesized oligonucelotides contain a series of codons which encode random combinations of amino acids and, in the 5' and 3' regions of the codons, contain sequences which encode antibody regions.

17. The process as claimed in claim 1, wherein the synthesized oligonucleotides contain a series of codons which encode random combinations of amino acids and, in the 5' and 3' regions of the codons, contain sequences which encode a spacer.

18. The process as claimed in claim 17, wherein the spacer contains proline residues.

19. The process as claimed in claim 1, wherein the microorganisms are transformed by electroporation.

20. The process as claimed in claim 1, wherein the nucleotide library comprises at least 10⁶.

21. Peptides possessing the amino acid sequences of the peptides SEQ ID NO: 4-17, which are prepared by at least one of the processes as claimed in claims 1-20.

22. Antibodies, which are produced using peptides as claimed in claim 21 which, where appropriate, are coupled to carrier proteins.

## Revendications

1. Procédé pour la production de peptides qui se lient à un partenaire de liaison spécifique préétabli, comprenant les étapes suivantes:
a) isolement ou synthèse d'oligonucléotides qui contiennent des régions dans lesquelles les séquences nucléotidiques varient de façon stochastique,
b) recombinaison des oligonucléotides avec des molécules de vecteur, de manière que les oligonucléotides soient insérés dans un gène, codé par un vecteur, qui exprime une protéine de l'enveloppe externe de microorganismes, un oligonucléotide étant inséré par molécule de vecteur, et il en résulte ainsi un grand nombre de molécules de vecteur qui, dans cette région, diffèrent quant à leur séquence nucléotidique (banque d'oligonucléotides),
c) transformation des micro-organismes par les molécules de vecteur recombinantes,
d) culture des micro-organismes transformés, dans des conditions permettant l'expression des séquences d'ADN recombinantes et la présentation des protéines recombinées correspondantes, à la surface des micro-organismes,
e) incubation des micro-organismes avec le partenaire de liaison spécifique préétabli, fixé à une phase solide, les micro-organismes qui ont exprimé le peptide recherché étant liés,
f) élimination des micro-organismes non liés,
g) multiplication des micro-organismes liés,
h) séparation et isolement des micro-organismes liés,
i) détermination des séquences d'aminoacides variées de façon stochastique, par séquençage des séquences d'ADN correspondantes ou par séquençage des peptides,
j) obtention des peptides recherchés, par isolement des protéines recombinées à partir des micro-organismes, ou synthèse chimique des peptides correspondants, caractérisé en ce que les micro-organismes sont choisis dans le groupe des bactéries et des cellules mammaliennes.

2. Procédé selon la revendication 1, dans lequel les organismes isolés dans les étapes e)-g) sont de nouveau mis en contact avec le partenaire de liaison, et l'étape de sélection et d'amplification est répétée.

3. Procédé selon la revendication 1, dans lequel les protéines recombinées à la surface des organismes et le récepteur choisi sont présents à des concentrations telles que seules des réactions de liaison monovalente sont possibles.

4. Procédé selon la revendication 1, dans lequel le micro-organisme est une bactérie.

5. Procédé selon la revendication 1, dans lequel le micro-organisme est une cellule mammalienne en suspension.

6. Procédé selon la revendication 4, dans lequel le micro-organisme est *E. coli*.

7. Procédé selon les revendications 4 et 6, dans lequel la protéine d'enveloppe est OmpA.

8. Procédé selon la revendication 1, dans lequel la phase solide dans l'étape e) est particulaire.

9. Procédé selon la revendication 8, dans lequel la phase solide est apte à l'attraction magnétique.

10. Procédé selon la revendication 1, dans lequel le vecteur est un plasmide.

11. Procédé selon la revendication 1, dans lequel les nucléotides contiennent des codons qui sont représentés par (NNK)ₓ, N représentant les nucléotides A, C, G, T, K représentant les nucléotides G, T, et x étant un nombre allant de 4 à 25.

12. Procédé selon la revendication 11, dans lequel x = 6.

13. Procédé selon la revendication 11, dans lequel x = 8 et les nucléotides ne codent que pour une fraction des octapeptides possibles.

14. Procédé selon la revendication 11, dans lequel x = 10 et les nucléotides ne codent que pour une fraction des décapeptides possibles.

15. Procédé selon la revendication 1, dans lequel les oligonucléotides contiennent une série de codons qui codent des combinaisons aléatoires d'aminoacides et contiennent, dans la région 5' et la région 3' des codons, des séquences qui ne sont pas non plus identiques à des séquences de protéine d'enveloppe.

16. Procédé selon la revendication 1, dans lequel les oligonucléotides synthétisés contiennent une série de codons qui codent des combinaisons aléatoires d'amino-acides et contiennent, dans la région 5' et la région 3' des codons, des séquences qui codent pour des régions d'anticorps.

17. Procédé selon la revendication 1, dans lequel les oligonucléotides synthétisés contiennent une série de codons qui codent des combinaisons aléatoires d'amino-acides et contiennent, dans la région 5' et la région 3' des codons, des séquences qui codent pour un espaceur.

18. Procédé selon la revendication 17, dans lequel l'espaceur contient des résidus proline.

19. Procédé selon la revendication 1, dans lequel les micro-organismes sont transformés par électroporation.

20. Procédé selon la revendication 1, dans lequel la banque nucléotidique comprend au moins 10⁶ nucléotides.

21. Peptides ayant les séquences d'aminoacides des peptides de SEQ ID n° 4 à 17, qui sont produits conformément à au moins l'un des procédés selon les revendications 1 à 20.

22. Anticorps, qui sont produits avec utilisation de peptides selon la revendication 21, éventuellement couplés à des protéines porteuses.
